# EUROPÄISCHE PATENTSCHRIFT

(11) **EP 2 123 230 B1**
(45) Veröffentlichungstag und Bekanntmachung des Hinweises auf die Patenterteilung: **21.03.2012**
(21) Anmeldenummer: 09167193.3
(22) Anmeldetag: 06.11.2004
(51) Int. Cl.: A61B 17/70, A61B 17/64

(54) **Federelement für eine Stabilisierungseinrichtung für Knochen und Herstellungsverfahren für das Federelement**
Spring element for a stabiliser device for bones and method for producing same
Elément de ressort pour un dispositif de stabilisation pour os et procédé de fabrication pour l'élément de ressort

(30) Priorität: 07.11.2003 DE 10351978; 07.11.2003 US 518469 P; 21.11.2003 US 523946 P; 03.03.2004 US 550182 P
(43) Veröffentlichungstag der Anmeldung: 25.11.2009
(62) Teilanmeldung aus: 04797689.9
(73) Patentinhaber: BIEDERMANN MOTECH GmbH, 78054 VS-Schwenningen (DE)
(72) Erfinder: Biedermann, Lutz, 78048, VS-Villingen (DE); Matthis, Wilfried, 79367, Weisweil (DE); Harms, Jürgen, 76227, Karlsruhe (DE)
(74) Vertreter: Prüfer & Partner GbR European Patent Attorneys

(56) Entgegenhaltungen:
- WO-A-03/047442
- FR-A- 2 717 370
- FR-A- 2 718 946
- US-A- 5 672 175
- US-A- 6 162 223
- US-A1- 2003 109 880

## Beschreibung

Die Erfindung betrifft ein Federelement für die Verwendung in der Wirbelsäulen- oder Unfallchirurgie, ein elastisches stabförmiges Element mit einem solchen Federelement zum Verbinden von Knochenverankerungselementen und ein Herstellungsverfahren für ein solches Federelement.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder über einem Stab verbundenen Knochenschrauben bestehen. Derartige starre Systeme erlauben keine Bewegung der relativ zu einander fixierten Knochenteile oder Wirbel.

Für bestimmte Indikationen ist jedoch eine dynamische Stabilisierung wünschenswert, bei der die zu stabilisierenden Knochenteile und Wirbel eine kontrollierte begrenzte Bewegung zu einander ausführen können. Eine Möglichkeit für die Realisierung der dynamischen Stabilisierungseinrichtung besteht in der Verwendung eines elastischen Elementes anstelle eines die Knochenverankerungselemente verbindenden starren Stabes.

Aus der US 2003/0109880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schrauben verbindenden Feder und eine solche Feder umfaßt. Die Feder selbst ist als Ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, daß die Feder auf Grund ihrer Elastizität dem Druck der Klemmschraube ausweicht und somit die Fixierung zwischen der Knochenschraube und der Feder gelockert wird. Ein weiterer Nachteil der Vorrichtung besteht darin, daß die Elastizität der Feder bei ansonsten gleichen Federeigenschaften von der Länge der Feder abhängt.

Aus der EP 0 669 109 B1 ist eine Stabilisierungseinrichtung zum Stabilisieren benachbarter Rückenwirbel bekannt, die zwei monoaxiale Pedikelschrauben und ein Band umfaßt, welches in den Aufnahmeteilen der Pedikelschrauben jeweils über eine Klemmschraube befestigt ist und welche ein auf das Band aufgezogenes druckfestes Stützelement beinhaltet. Mit einer derartigen Stabilisierungseinrichtung wird jedoch keine Stabilisierung gegen Torsion erreicht. Wie bei der weiter oben beschriebenen Stabilisierungseinrichtung ist die Elastizität der Verbindung der beiden Knochenverankerungselemente bei ansonsten gleichen Eigenschaften der Federelemente abhängig von der Länge der Federelemente bzw. von dem Abstand der Knochenverankerungselemente.

Aus der US 6,162,223 ist eine Fixationseinrichtung für ein Gelenk, z.B. für ein Handgelenk oder ein Kniegelenk, bekannt, bei der ein an seinen Enden mit Knochenverankerungselementen verbundener Fixationsstab zweiteilig ausgebildet ist, wobei die zwei Teile des Fixationsstabs über ein flexibles Kupplungsteil miteinander verbunden sind und wobei die Fixa-tionsstäbe um das Kupplungsteil außerhalb des Körpers angebracht sind. Die beiden Teile des Fixationsstabs sind mit dem Kupplungsteil nicht fest verbunden, sondern können sich entlang einer Bohrung in dem Kupplungsteil frei bewegen. Der Durchmesser des Kupplungsteil ist auf Grund der Art der Verbindung mit dem zweiteiligen Fixationsstab immer größer als der Durchmesser des Fixationsstabs. Diese bekannte Fixations-einrichtung ist auf Grund ihres komplizierten und voluminösen Aufbaus zum inneren Einsatz an der Wirbelsäule oder anderen Knochen nicht geeignet.

WO-A-03/07442 offenbart ein elastiches Element mit den Merkmalen des Oberbegriffs das Patentanspruchs 1. FRA- 2717370 und FR-A-2718946 offenbarenähnliche elastische Elemente zur Verwendung in einer Stabilisierungseinrichtung für Wirbel.

Es ist Aufgabe der Erfindung, ein Federelement bereit zu stellen, das einfach und kompakt gebaut, sowie leicht zu handhaben bei gleichzeitig hoher Sicherheit im Einsatz ist, und welches mit anderen Elementen in möglichst vielfältiger Weise zu einer dynamischen Stabilisierungseinrichtung für miteinander zu verbindende Wirbel oder Knochen kombiniert werden kann. Ferner ist es Aufgabe der Erfindung, ein Herstellungsverfahren für das Federelement bereit zu stellen.

Die Aufgabe wird gelöst durch ein Federelement nach dem Patentanspruch 1 und ein Verfahren zum Herstellen eines solchen Federelementes.

Weiterbildungen der Erfindung sind in den Unteransprüchen angegeben.

Die Erfindung weist den Vorteil auf, daß ein Federelement wahlweise mit starren stabförmigen Elementen verschiedener Länge zu einem elastischen stabförmigen Element kombinierbar ist oder mit verschiedenen Schäften und/oder Köpfen zu einer Knochenschraube mit elastischen Eigenschaften kombinierbar ist. Der Federstab bzw. die Knochenschraube weisen dann in Abhängigkeit von dem verwendetem Federelement vorgegebene elastische Eigenschaften wie eine bestimmte Kompressions- und Extensionsfähigkeit in axialer Richtung, sowie eine bestimmte Biege- und Torsionssteifigkeit auf.

Insbesondere kann das Federelement mit stabförmigen Bauteilen verschiedener Dicke oder mit in der Wirbelsäulen- und/oder Unfallchirurgie zu verwendenden Platten unterschiedlicher Form und Länge verbunden werden.

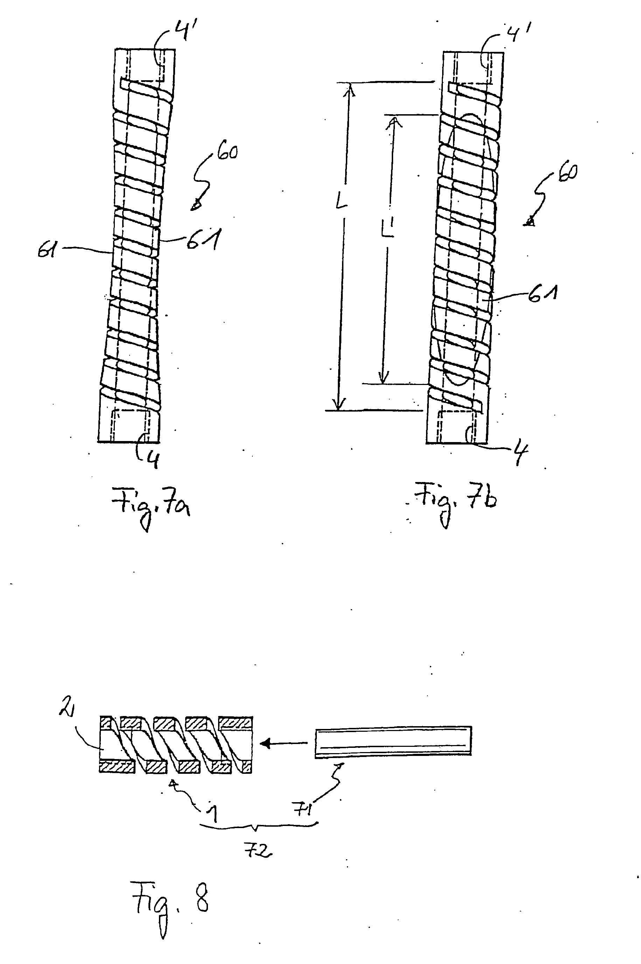

Weitere Merkmale und Zweckmäßigkeiten ergeben sich aus der Beschreibung von Ausführungsbeispielen anhand der Figuren. Nur Figur 8 zeigt ein Ausfuhrüngsbeispiel nach der Erfindung. Von den Figuren zeigen:
- Fig. 1a: eine Seitenansicht einer ersten Ausführungsform des Federelementes;
- Fig. 1b: eine Schnittdarstellung des Federelementes aus Fig. 1a;
- Fig. 2a: ein erstes Anwendungsbeispiel des Federelements in Form eines stabförmigen Elements;
- Fig. 2b: eine Abwandlung von Fig. 2a;
- Fig. 3a: ein Knochenverankerungselement mit dem Federelement aus Fig. 1a;
- Fig. 3b: eine Schnittdarstellung eines _Teils der Ausführungs- form von Fig. 3a mit einer Weiterbildung;
- Fig. 3c: eine Schnittdarstellung des Teils von Fig. 3b entlang der Linie A-A;
- Fig. 4: eine Stabilisierungseinrichtung bestehend aus zwei dreiteiligen Knochenverankerungselementen und einem stabförmigen Element, die jeweils ein Federelement aufweisen;
- Fig. 5: eine Seitenansicht einer zweiten Ausführungsform des Federelementes;
- Fig. 6: eine Seitenansicht einer dritten Ausführungsform des Federelementes;
- Fig. 7a: eine Seitenansicht eines Federelementes nach einer vierten Ausführungsform;
- Fig. 7b: eine um 90° gedrehte Seitenansicht des Federelementes aus Fig. 7a;
- Fig. 8: eine Schnittdarstellung eines Federelementes nach ei- ner fünften Ausführungsform;
- Fig. 9: ein Federelement nach einer sechsten Ausführungsform;
- Fig. 10: ein Federelement nach einer siebten Ausführungsform;
- Fig. 11a: eine Explosionsdarstellung eines Verbindungselementes, das aus einem stabförmigen Element, einem Federelement und einer Platte besteht;
- Fig. 11b: eine Schnittdarstellung der Platte aus Fig. 11a ent- lang der Linie A-A;
- Fig. 12: ein Anwendungsbeispiel der Platte aus Fig. 11a und 11b, bei dem die Platte und das mit der Platte über ein Federelement verbundene stabförmige Element mit Knochenverankerungselementen jeweils in Wirbeln veran- kert ist;
- Fig. 13a: eine Anwendung eines Federelementes in einer dynamischen Stabilisierungseinrichtung für einen Beckenknochen;
- Fig. 13b: eine Schnittdarstellung eines bei der Stabilisierungs- einrichtung aus Fig. 13a verwendeten Knochenveranke- rungselementes;
- Fig. 14: eine Anwendung des Federelementes in einem Fixateur Externe mit Schanzschrauben, die über stabförmige Ele- mente miteinander verbunden sind; und
- Fig. 15: ein mittels Drahterodieren, Laserbearbeitung oder Was- serstrahlbearbeitung hergestelltes Federelement.

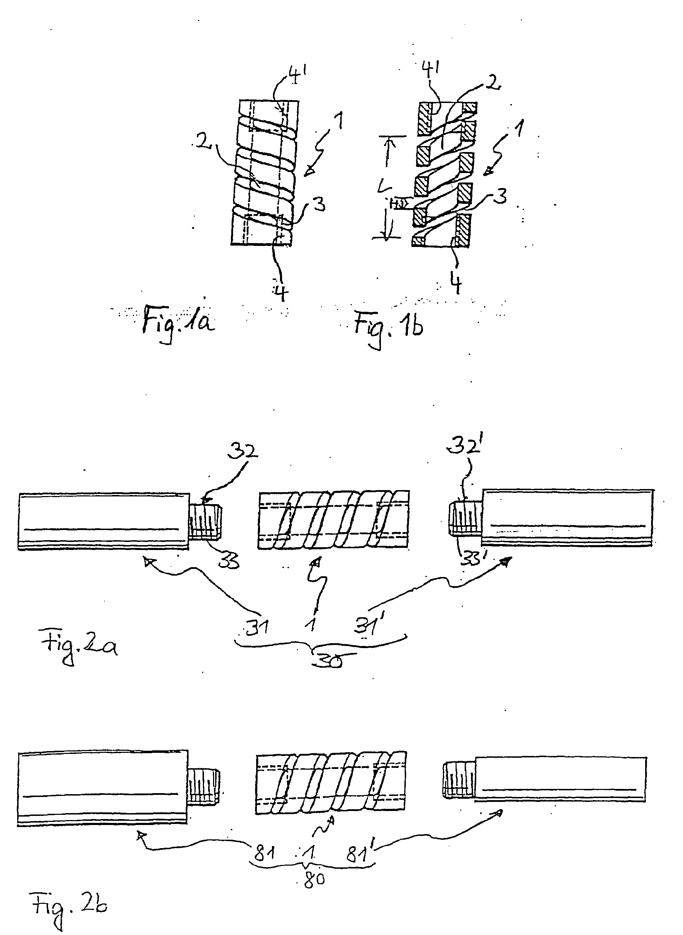

In den Figuren 1a und 1b ist ein Federelement 1 nach einer ersten Ausführungsform dargestellt.

Das Federelement 1 besteht aus einem zylindrischen Rohr mit einer durchgehenden koaxialen Bohrung 2 und einer spiralförmig mit einer vorbestimmten Steigung und über eine vorbestimmte Länge in Richtung der Zylinderachse in der Wandung verlaufenden Ausnehmung 3, die in radialer Richtung in die Bohrung 2 mündet. Dadurch ist eine Schraubenfeder gebildet. Die Länge der spiralförmigen Ausnehmung in Richtung der Zylinderachse, die Höhe der Ausnehmung, die Steigung der Spirale und der Durchmesser der koaxialen Bohrung sind so gewählt, daß eine gewünschte Steifigkeit der Schraubenfeder gegenüber axialen Kräften, Biegekräften und Torsionskräften, die auf das Federelement wirken, gegeben ist. Angrenzend an seine beiden freien Enden weist das Federelement 1 jeweils ein sich über eine vorbestimmte Länge erstreckendes Innengewinde 4, 4' auf. Der Außendurchmesser des Federelementes ist der jeweiligen Anwendung entsprechend gewählt.

In einem ersten, in Fig. 2a gezeigten Anwendungsbeispiel ist das Federelement 1 Bestandteil eines elastischen stabförmigen Elements 30. Das elastische stabförmige Element 30 besteht aus dem Federelement 1 und zwei zylindrischen Stabschnitten 31, 31', die an ihrem Ende jeweils einen zylindrischen Ansatz 32, 32' mit einem Außengewinde 33, 33' aufweisen, das mit dem Innengewinde 4, 4' des Federelements 1 zusammenwirkt. Die Stababschnitte und das Federelement weisen in diesem Ausführungsbeispiel im wesentlichen denselben Außendurchmesser auf. Die Länge der Stababschnitte 31, 31' und des Federelements 1 sind unabhängig voneinander im Hinblick auf eine gewünschte Anwendung wählbar. Das stabförmige Element dient beispielsweise zur Verbindung von Pedikelschrauben an der Wirbelsäule. Das so gebildete stabförmige Element 30 nimmt durch die elastischen Eigenschaften des Federelementes 1 in vorgegebenen Umfang Kompressions-, Extensions-, Biege- und Torsionskräfte auf.

In Fig. 2b ist ein elastisches stabförmiges Element 80 gezeigt, das sich von dem elastischen stabförmigen Element 30 dadurch unterscheidet, daß ein erster starrer Stababschnitt 81 einen größeren Außendurchmesser aufweist als das Federelement 1 und der zweite starre Stababschnitt 81' einen kleineren Außendurchmesser aufweist, als das Federelement 1. Alternativ können auch beide Stababschnitte einen größeren oder einen kleineren Durchmesser haben als das Federelement.

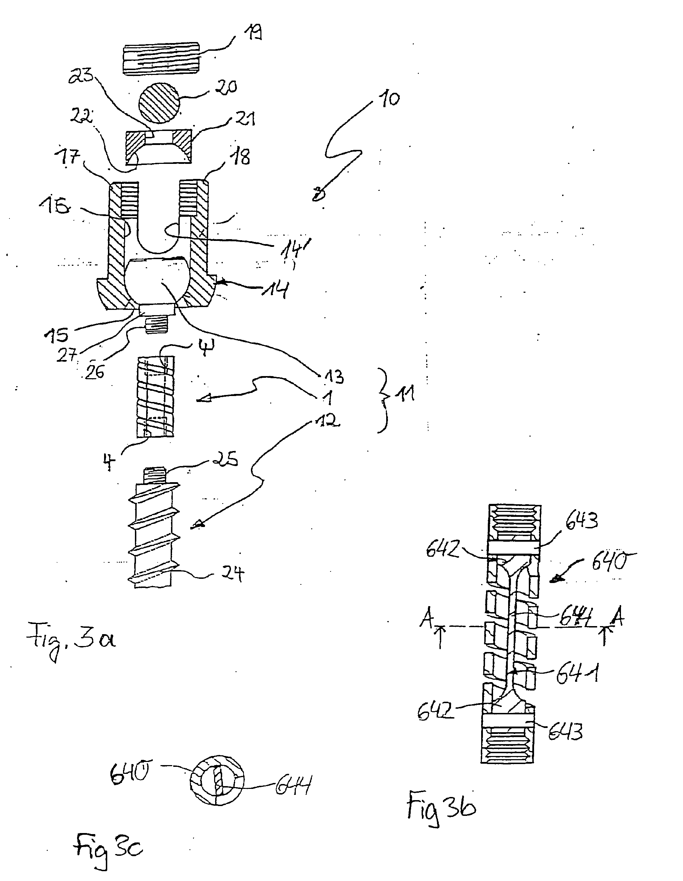

Fig. 3a zeigt ein zweites Anwendungsbeispiel des Federelementes. Das Federelement 1 ist hier Bestandteil eines Knochenverankerungselements 10, das als Polyaxial-Knochenschraube ausgebildet ist. Die Polyaxialknochenschraube weist ein Schraubenelement 11 auf, welches aus dem Federelement 1, einem Schaft 12 mit einer nicht dargestellten Spitze und einem Schraubenkopf 13 besteht.

Der Schaft 12 weist ein Knochengewinde 24 zum Einschrauben in den Knochen und einen zylinderförmigen Ansatz 25 mit einem Außengewinde auf, das mit dem Innengewinde 4 des Federelementes 1 zusammenwirkt.

Der Schraubenkopf 13 weist einen zylinderförmigen Abschnitt 27 und daran angrenzend wie der Schaft 12 einen zylinderförmigen Ansatz 26 mit einem Außengewinde auf, das mit dem Innengewinde 4' des Federelementes 1 zusammenwirkt.

Das Schraubenelement 11 ist in einen Aufnahmeteil 14 in unbelastetem Zustand schwenkbar gehalten. Das Aufnahmeteil 14 ist im wesentlichen zylindrisch ausgebildet und weist an seinem einen Ende eine axialsymmetrisch ausgerichtete erste Bohrung 15 auf, deren Durchmesser größer als der des Schafts 12 und kleiner als der des Schraubenkopfs 13 ist. Ferner weist das Aufnahmeteil 14 eine koaxiale zweite Bohrung 16 auf, die auf dem der ersten Bohrung 15 gegenüberliegenden Ende offen ist und deren Durchmesser so groß ist, daß das Schraubenelement durch das offene Ende mit dem Schaft durch die erste Bohrung 15 hindurchführbar ist, bis der Schraubenkopf 13 am Rand der ersten Bohrung 15 anliegt. Das Aufnahmeteil 14 weist eine sich vom freien Ende in Richtung der ersten Bohrung 15 erstreckende U-förmige Ausnehmung 14' auf, durch die zwei freie Schenkel 17, 18 gebildet sind. In einem Bereich angrenzend an ihr freies Ende weisen die Schenkel 17, 18 ein Innengewinde auf, welches mit einem entsprechenden Außengewinde einer Innenschraube 19 zum Fixieren eines Stabs 20 zusammenwirkt.

Es ist ferner ein Druckelement 21 zum Fixieren des Schraubenkopfs 13 in dem Aufnahmeteil 14 vorgesehen, das so ausgebildet ist, daß es an seiner dem Schraubenkopf 13 zugewandten Seite eine sphärische Ausnehmung 22 aufweist, deren Radius im wesentlichen gleich dem Radius des kugelsegmentförmigen Abschnitts des Schraubenkopfes 13 ist. Der Außendurchmesser des Druckelements 21 ist so gewählt, daß das Druckelement 21 in dem Aufnahmeteil 14 zu dem Schraubenkopf 13 hin verschiebbar ist. Das Druckelement 21 weist ferner eine koaxiale Bohrung 23 für den Zugriff auf eine nicht dargestellte Ausnehmung in dem Schraubenkopf 13 zum Ineingriffbringen mit einem Einschraubwerkzeug auf.

Im Betrieb wird in das Innengewinde 4 des Federelementes 1 der Schaft 12 mit seinem zylinderförmigen Ansatz 25 und in das Innengewinde 4' der Schraubenkopf 13 mit seinem zylinderförmigen Ansatz 26 eingeschraubt, um ein Schraubenelement 11 zu bilden. Danach wird das so gebildete Schraubenelement 11 mit dem Schaft 12 voran durch die zweite Öffnung in das Aufnahmeteil 14 eingeführt, bis der Schraubenkopf 13 am Rand der ersten Bohrung 15 anliegt. Anschließend wird das Druckelement 21 mit der sphärischen Ausnehmung voran durch die zweite Öffnung 16 in das Aufnahmeteil 14 eingeführt. Dann wird das Schraubenelement 11 in den Knochen bzw. Wirbel eingeschraubt. Schließlich wird der Stab 20 in das Aufnahmeteil 14 zwischen die beiden Schenkel 17 und 18 eingelegt, die Winkelstellung des Aufnahmeteils relativ zu dem Schraubenelement justiert und mit der Innenschraube 19 fixiert. Durch den elastischen Abschnitt werden Bewegungen um die Ruhelage in begrenzter Weise ermöglicht.

Die Polyaxialschraube ist nicht auf die zuvor beschriebene Ausführungsform beschränkt, sondern kann auch jede beliebige andere Polyaxialschraube mit einem wie oben beschriebenen dreiteiligen Schraubenelement sein. So kann die erste Bohrung 15 bei dem in Fig. 3a dargestellten Anwendungsbeispiel auch geringer im Durchmesser sein als der Schaft 12, wenn im Betrieb zuerst der Schraubenkopf 13 mit seinem zylinderförmigen Ansatz 26 voran durch die zweite Bohrung 16 in das Aufnahmeteil 14 eingeführt wird, bevor das Federelement 1 und der Schaft 12 an den Schraubenkopf 12 angeschraubt wird. Es ist in diesem Fall ausreichend, wenn die erste Bohrung 15 einen größeren Durchmesser hat als der zylinderförmige Ansatz 26 und als der zylinderförmige Abschnitt 27. Alternativ kann der Schraubenkopf 13 auch ohne den zylinderförmigen Abschnitt 27 ausgebildet sein. Die Bohrung muss dann lediglich so groß sein, dass der Ansatz 26 hindurchführbar ist.

Das Aufnahmeteil kann aber auch derart gestaltet sein, dass das Schraubenelement von unten einführbar ist und über ein Druckelement im Aufnahmeteil verklemmt wird. Die in Fig. 3a gezeigte Bohrung 15 ist dann größer als der Durchmesser des Schraubenkopfes 13.

Die Stabfixierung ist nicht auf die in Fig. 3a gezeigte Innenschraube beschränkt, sondern es kann zusätzlich eine Außenmutter vorgesehen sein, oder jede bekannte Art der Stabfixierung kann eingesetzt werden.

Wenn das Federelement 1 wenigstens teilweise über die Knochenoberfläche hervorsteht kann das Federelement 1 Biegekräfte, sowie Zug- und Druckkräfte aufnehmen. Steht das Federelement nicht mehr über die Knochenoberfläche hervor, kann das Schraubenelement 11 trotzdem bei einer Bewegung des Knochens bzw. Wirbels etwas nachgeben. Damit wird verhindert, dass ungünstige Spannungen auftreten.

Fig. 3b zeigt eine Weiterbildung des Federelements 1 von Fig. 3a. Das Federelement 640 gemäß Fig. 3b weist in seinem Inneren einen Kern 641 auf. Der Kern 641 ist an seinen Enden 642 zylindrisch ausgebildet mit einem Durchmesser, der so bemessen ist, daß der Kern in den Hohlraum des elastischen Abschnitts 640 einschiebbar ist. Die zylindrischen Abschnitte 642 und der elastische Abschnitt 640 weisen querverlaufende Bohrungen auf, in die Stifte 643 zum Befestigen des Kerns geschoben sind. Zwischen den zylindrischen Enden weist der Kern einen Abschnitt 644 mit im wesentlichen rechteckigem Querschnitt auf, wie insbesondere aus Fig. 3c ersichtlich ist. In einer Abwandlung hat der Kern einen anderen Querschnitt, wie z.B. oval oder asymmetrisch. Der Kern erlaubt die Einstellung der Biegesteifigkeit und/oder der Torsionssteifigkeit des elastischen Abschnitts. Die Steifigkeit des elastischen Abschnitts gegenüber einer Biegung in einer bestimmten Richtung hängt von der Orientierung des Kerns innerhalb des elastischen Abschnitts ab. Vorzugsweise ist der Kern aus einem Material mit geringerer Steifigkeit verglichen mit dem Material des elastischen Abschnitts ausgebildet. Die gezeigte Befestigung des Kerns ist nur beispielhaft dargestellt.

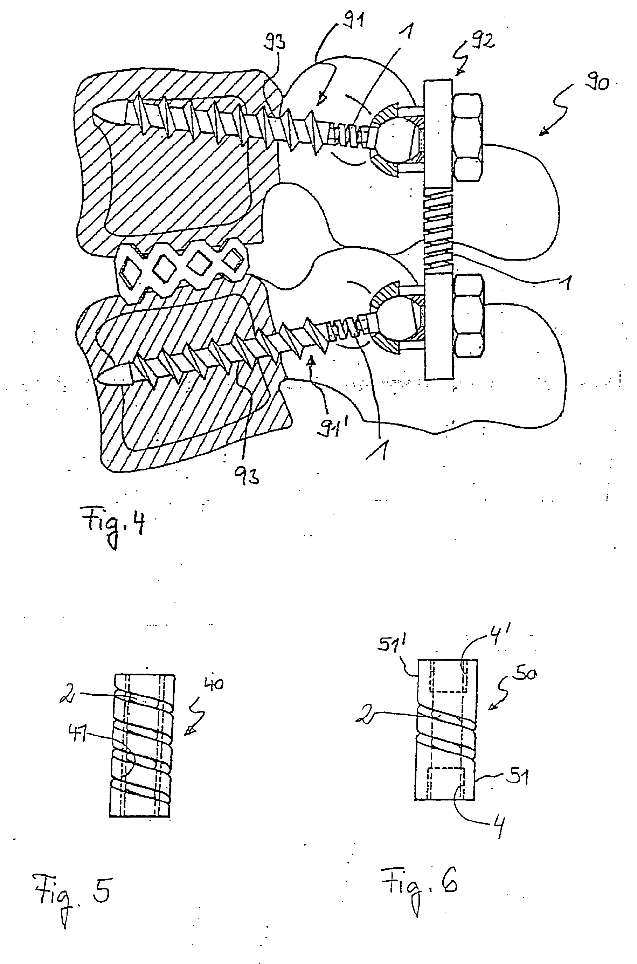

In Fig. 4 ist eine Stabilisierungsvorrichtung 90 für die Wirbelsäule dargestellt, wobei zwei Knochenverankerungselemente 91, 91' mit Schraubenelementen 93 und ein elastisches stabförmiges Element 92 jeweils mit einem erfindungsgemäßen Federelement 1 zur Verbindung der beiden Knochenverankerungselemente verwendet wird. Durch die Mehrteiligkeit des elastischen stabförmigen Elementes und des Schraubenelementes ist es möglich, durch die Kombination von nur wenigen Grundelementen Stabilisierungsvorrichtungen 90 mit den unterschiedlichsten Eigenschaften zu erhalten. Die Stabilisierungsvorrichtung muß nicht notwendigerweise Knochenverankerungselemente mit einem Federelement und ein stabförmiges Element mit dem Federelement beinhalten. Je nach Anwendungsgebiet ist es auch möglich, nur ein stabförmiges Element mit Federelement und Knochenverankerungselemente mit starren Schraubenelementen vorzusehen.

In Fig. 5 ist ein Federelement 40 nach einer zweiten Ausführungsform dargestellt. Das Federelement 40 nach der zweiten Ausführungsform unterscheidet sich von dem Federelement 1 nach der ersten Ausführungsform nur darin, dass anstelle der beiden Innengewinde 4, 4' ein über die ganze Länge des Federelementes ausgebildetes Innengewinde 41 vorgesehen ist.

In Fig. 6 ist ein Federelement 50 nach einer dritten Ausführungsform dargestellt. Es weist im Unterschied zur ersten und zur zweiten Ausführungsform starre Endabschnitte 51 und 51' bzw. eine im Unterschied zu den vorherigen Ausführungsformen verringerte Anzahl von Spiralwindungen auf. Dadurch lässt sich die Elastizität des Federelementes unabhängig von der Länge des Federelementes gestalten.

In den Figuren 7a und 7b ist ein Federelement 60 nach einer vierten Ausführungsform dargestellt, das im Unterschied zu den vorgehenden Ausführungsformen zwar um 180° gegeneinander versetzte konkav zur Mittenachse hin angeformte Bereiche 61 aufweist. Die Länge L' der Bereiche 61 in Richtung der Mittenachse ist maximal gleich der Länge L der Spirale und der Krümmungsradius der angeformten Bereiche 61 ist derart, daß die Windungen der Schraubenfeder nicht durchbrochen sind. Durch diese Ausbildung ist das Federelement in einer Richtung senkrecht zur Mittenachse tailliert ausgebildet und hat somit eine geringere Steifigkeit in dieser Richtung. Damit hat das Federelement eine orientierte Steifigkeit, die für bestimmte Anwendungen zweckmäßig ist.

Ein in Fig. 8 dargestelltes Federelement 72 nach einer fünften Ausführungsform weist zusätzlich zu dem Federelement 1 nach der ersten Ausführungsform einen in die Bohrung eingeschobenen stabförmigen Kern 71 auf. Der Kern kann einerseits als Anschlag bei auf das Federelement 72 ausgeübten Druckkräften dienen. Andererseits kann mit dem Kern 71 die Steifigkeit des Federelementes 72 in bezug auf Biegekräfte erhöht werden.

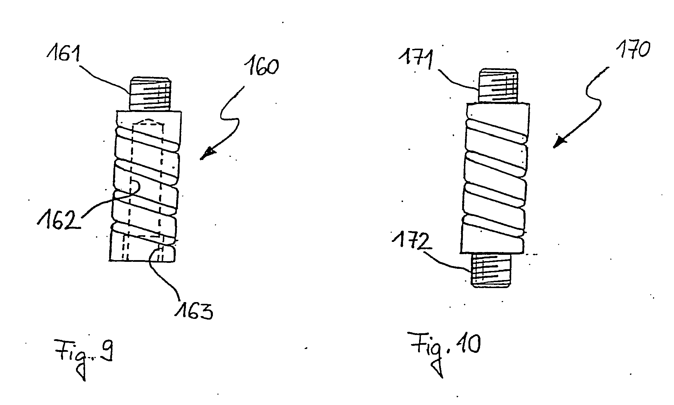

Ein in Fig. 9 dargestelltes Federelement 160 nach einer sechsten Ausführungsform weist an seinem einen Ende, anstelle einer Bohrung mit einem Innengewinde wie bei der ersten Ausführungsform, einen zylindrischen Ansatz 161 mit einem Außengewinde auf. Entsprechend ist das mit diesem Ende des Federelements zu verbindende Element mit einer Bohrung mit einem entsprechenden Innengewinde ausgebildet. Das andere Ende des Federelements ist mit einer Sackbohrung 162 versehen, in der an das Ende des Federelementes angrenzend wie bei den zuvor beschriebenen Ausführungsformen ein Innengewinde 163 ausgebildet ist.

Ein in Fig. 10 dargestelltes Federelement 170 nach einer siebten Ausführungsform weist an seinen beiden Enden jeweils einen zylinderförmigen Ansatz 171, 172 mit einem Außengewinde auf. In einer Abwandlung der ersten bis fünften Ausführungsform weist das Federelement keine durchgehende Bohrung auf.

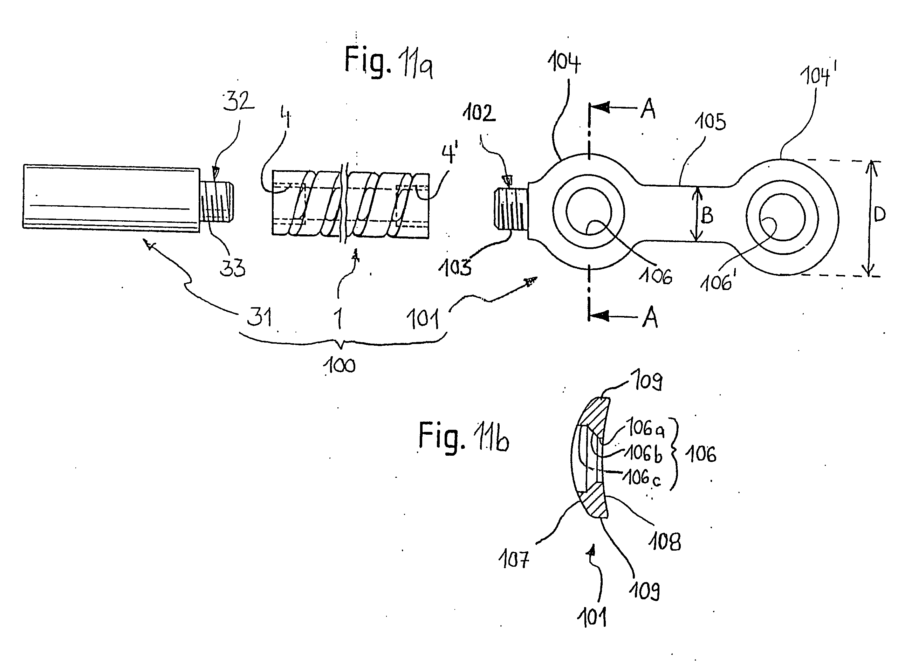

Als weiteres Anwendungsbeispiel für das Federelement 1 ist in Fig. 11a die Explosionsdarstellung eines Verbindungselementes 100 zu sehen, das aus einem stabförmigen Element 31, einem Federelement 1 und einer Platte 101 besteht. Das stabförmige Element 31 weist einen zylindrischen Ansatz 32 mit einem Außengewinde 33 zum Einschrauben in das an das eine Ende des Federelementes 1 angrenzende Innengewinde 4 auf. Ebenso weist die Platte 101 einen zylindrischen Ansatz 102 mit einem Außengewinde 103 zum Einschrauben in das an das andere Ende des Federelementes 1 angrenzende Innengewinde 4' auf. Die Platte besteht aus zwei in der Draufsicht kreisförmigen Abschnitten 104, 104', die über einen Steg 105 miteinander verbunden sind. Die Breite B des Steges 105 ist geringer als der Durchmesser D der kreisförmigen Abschnitte 104, 104'. Koaxial zu den kreisförmigen Abschnitten sind zwei Bohrungen 106, 106' für Senkschrauben durch die Platte vorgesehen. Wie in Fig. 11b zu sehen weist die erste Seite 107 der Platte eine konkave Krümmung auf während die zweite Seite 108 der Platte eine konvexe Krümmung zum Anliegen dieser Seite an einen Knochen aufweist. Durch die unterschiedlichen Krümmungsradien der beiden Seiten 107, 108 der Platte 101 verjüngt sich die Platte 101 zu den seitlichen Rändern 109 hin. Dadurch kann die Platte stabil und gleichzeitig raumsparend sein. Die Bohrungen 106, 106' weisen wie in Fig. 11b zu sehen an die zweite Seite 108 angrenzend eine Öffnung 106a, daran angrenzend einen konisch geformten ersten Abschnitt 106b und einen an den ersten Abschnitt und die erste Seite 107 angrenzenden zweiten Abschnitt 106c auf. Durch diese Form der Bohrungen 106, 106' sind diese zum Aufnehmen von Senkschrauben geeignet ausgebildet. Die Form der Bohrungen 106, 106' kann auch von der zuvor beschriebenen Form abweichen, soweit sie zur Aufnahme einer Senkschraube geeignet ist.

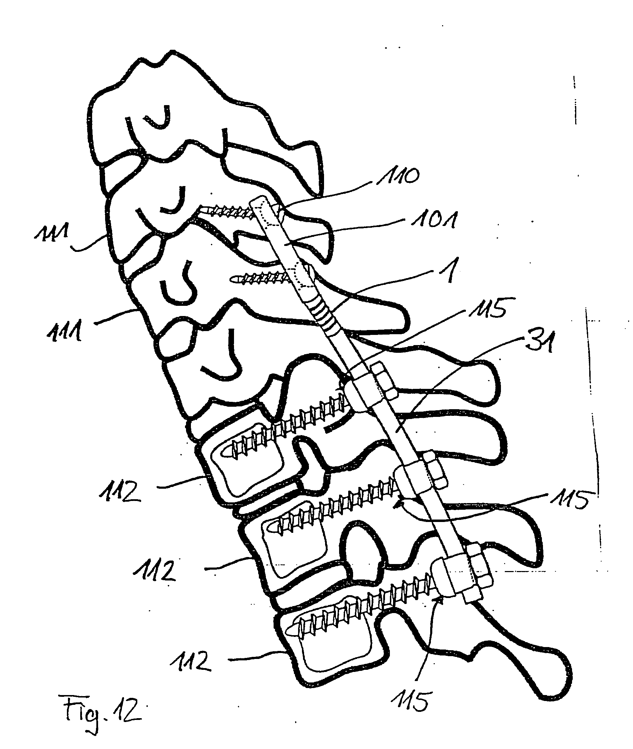

In Fig. 12 ist ein Anwendungsbeispiel für das Verbindungselement 100 aus Fig. 11a, bei dem die Platte 101 von der posterioren Seite mit zwei Knochenschrauben 110 an zwei Wirbeln 111 der Halswirbelsäule fixiert ist, und bei dem das mit der Platte über ein Federelement 1 verbundene stabförmige Element 31 mit drei Knochenverankerungselementen 115 in Wirbeln 112 der Brustwirbelsäule verankert ist.

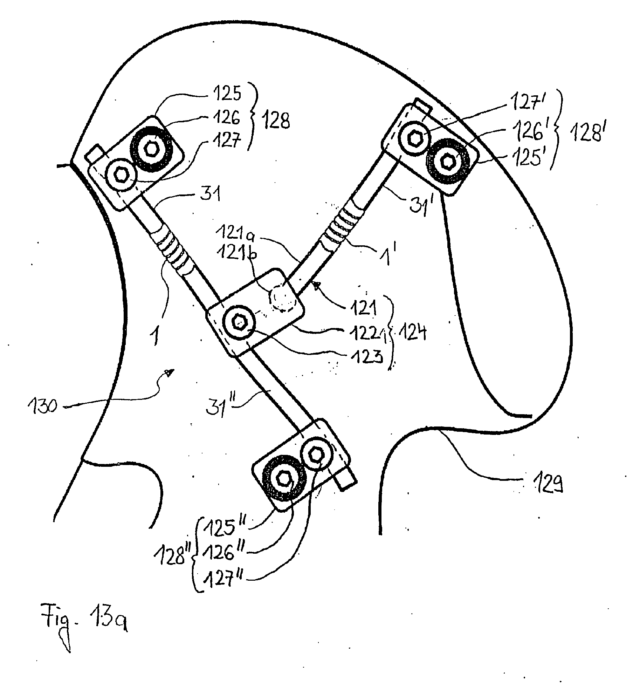

Ein weiteres Anwendungsbeispiel, bei dem das Federelement 1 in einer dynamischen Beckenstabilisierungsseinrichtung 130 verwendet wird, ist in Fig. 13a dargestellt. Die dynamische Beckenstabilisierungseinrichtung besteht aus Knochenverankerungselementen 128, 128', 128", die mit stabförmigen Elementen 31, 31', 31" und Federelementen 1, 1' miteinander verbunden sind.

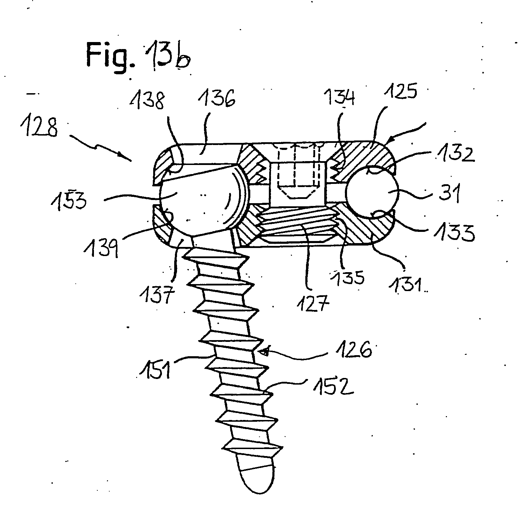

Das in Fig. 13b dargestellte Knochenverankerungselement 128 besteht wie die beiden anderen Knochenverankerungselemente 128', 128" aus zwei Hälften 125, 131, die mit einer in ein Gewinde 134 in der ersten Hälfte 125 und in eine Gewinde 135 in der zweiten Hälfte 131 eingreifenden Schraube 127 miteinander verschraubt sind. In der Draufsicht in Fig. 13a ist davon nur die eine obere Hälfte 125 sichtbar. Zwischen den beiden zuvor erwähnten Hälften 125, 131 ist das stabförmige Element 31 in einer Ausnehmung 132 in der ersten Hälfte 125 und in einer Ausnehmung 133 in der zweiten Hälfte 131 zwischen den beiden Hälften eingeklemmt, sodass das Knochenverankerungselement 128 fest mit dem stabförmigen Element 31 verbunden ist. Weiter sind in den beiden Hälften 125, 131 jeweils eine Bohrung 136 bzw. 137 vorgesehen, die im montierten Zustand koaxial ausgerichtet sind. An die Bohrung 136 angrenzend ist eine sphärische Ausnehmung 138 und an die Bohrung 137 angrenzend ist eine sphärische Ausnehmung 139 ausgebildet, die der Aufnahme einer Knochenschraube 126 dienen. Die Knochenschraube 126 weist einen schaftförmigen Abschnitt 151 mit einem Außengewinde 152 zum Einschrauben in den Knochen und einen kugelsegmentförmigen Kopfabschnitt 153 mit einem Radius der im wesentlichen gleich dem Radius der sphärischen Ausnehmungen 138, 139 ist.

Das Verbindungselment 124 besteht wie das Knochenverankerungselement aus zwei Hälften 122, von denen in der Draufsicht nach Fig. 13a nur eine zu sehen ist. Zwischen diese beiden zuvor erwähnten Hälften 122 ist das stabförmige Element 31 in einer Ausnehmung geführt eingeklemmt, sodass das Verbindungselement 124 fest mit dem stabförmigen Element 31 verbunden ist.

Das Stabelement 121 besteht aus einem kugelförmigen Kopfabschnitt 121b und einem Schaftabschnitt 121a. Der Kopfabschnitt 121b wird zwischen den beiden Hälften 122 in einer nicht dargestellten Ausnehmung festgeklemmt und ist so in einer bestimmten Schwenkstellung fixierbar mit den beiden Hälften 122 verbunden. An seinem dem Kopfabschnitt 121b gegenüberliegenden Ende weist der Schaftabschnitt 121a einen zylinderförmigen Ansatz (nicht dargestellt) mit einem Außengewinde auf, das in das Innengewinde (nicht dargestellt) des Federelementes 1' eingeschraubt ist.

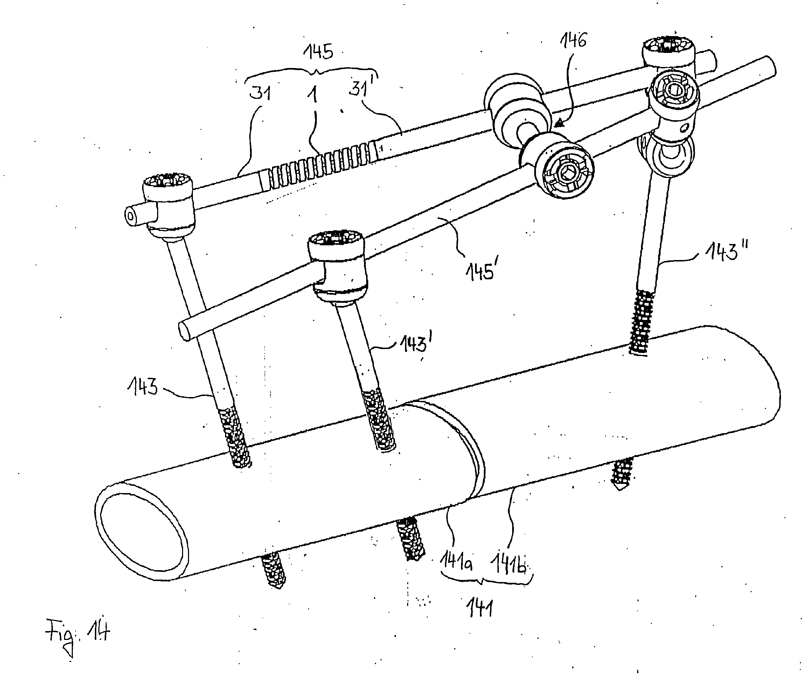

In Fig. 14 ist ein weiteres Anwendungsbeispiel für das Federelement 1 dargestellt. Das Federelement 1 ist hier Teil eines Fixateur Externe zum Stabilisieren eines aus zwei Teilen 141a und 141b bestehenden Knochens 141.

In das erste Teil 141a des Knochens 141 sind eine erste und eine zweite Schanzschraube 143, 143' und in den zweiten Teil des Knochens 141b des Knochens 141 ist eine dritte Schanzschraube 143" eingeschraubt. Die erste Schanzschraube 143 und die zweite Schanzschraube 143' sind in an sich bekannter Art und Weise über einen ersten Stab 145 bzw. einen zweiten Stab 145' mit der dritten Schanzschraube 143" verbunden. Der erste und der zweite Stab 145, 145' sind zusätzlich mit einem hier nicht näher beschriebenen Kopplungselement 146 in an sich bekannter Art und Weise fest miteinander verbunden.

Der erste Stab 145 ist dreiteilig aus zwei wie weiter oben mit Bezug auf Fig. 2 beschriebenen stabförmigen Elementen 31, 31' und einem erfindungsgemäßen Federelement 1 ausgebildet. Dabei ist das erste stabförmige Element über eine Schraubverbindung fest mit dem einen Ende des Federelementes 1 und das zweite stabförmige Element über eine Schraubverbindung fest mit dem anderen Ende des Federelementes 1 verbunden.

Durch die dynamische Stabilisierung des Knochens 141 werden kleinere Bewegungen der beiden Knochenenteile 141a und 141b untereinander erlaubt. Diese kleineren Bewegungen führen zu einer wünschenswerten Stimulation für das Zusammenwachsen der beiden Knochenteile 141a, 141b.

Je nach Anwendungsgebiet kann auch eine Schanzschraube des Fixateur Externe ein derartiges Federelement als Teil seines Schaftes aufweisen.

Als weitere Anwendungsbeispiele für das Federelement sind zum Beispiel der Einsatz in einer Knochenschraube, die nur einen Schaft mit einem Knochengewinde und eine Spitze sowie einem Kopf aufweist, wie sie üblicherweise zur Verbindung mit Stabilisierungsplatten verwendet werden. Ein weiteres Anwendungsbeispiel des Federelements ist der Einsatz in einer Monoaxial-Knochenschraube zum Verbinden mit einem Stab.

Zur Herstellung eines Federelementes 1 mittels Fräsen geht man von einem Zylinder mit einem vorbestimmten Aussendurchmesser aus einem biokompatiblen Material, wie z.B. Titan, aus und fräst mit einem dünnen Scheibenfräser entlang einer Spirale, deren Hauptachse kollinear zu der Hauptachse des Zylinders ist eine Ausnehmung 3. Anschließend wird entlang der Hauptachse des Zylinders über die gesamte Länge des Zylinders eine Bohrung 2 derart gebildet, dass die spiralförmige Ausnehmung 3 in die Bohrung 2 mündet. Für die Stabilität des Federelementes 1 ist der Auslauf der Spirale am Übergang zwischen dem Spiralabschnittes und dem endseitigen Abschnitt des Federelementes von größter Bedeutung. Daher ist es notwendig, mit einem Fingerfräser den Auslauf der Spirale an beiden Enden der Spirale derart nachzubearbeiten, dass die scharfe Kante an der Innenseite der Bohrung entfernt wird. Dazu wird der Auslauf mit einem Fingerfräser in einem Winkel tangential zur Spiralkontur gefräst. Im Anschluß daran wird das Bauteil innen und außen entgratet. Schließlich wird in den zwei Endabschnitten der Bohrung 2 jeweils ein Innengewinde 4, 4' gebildet.

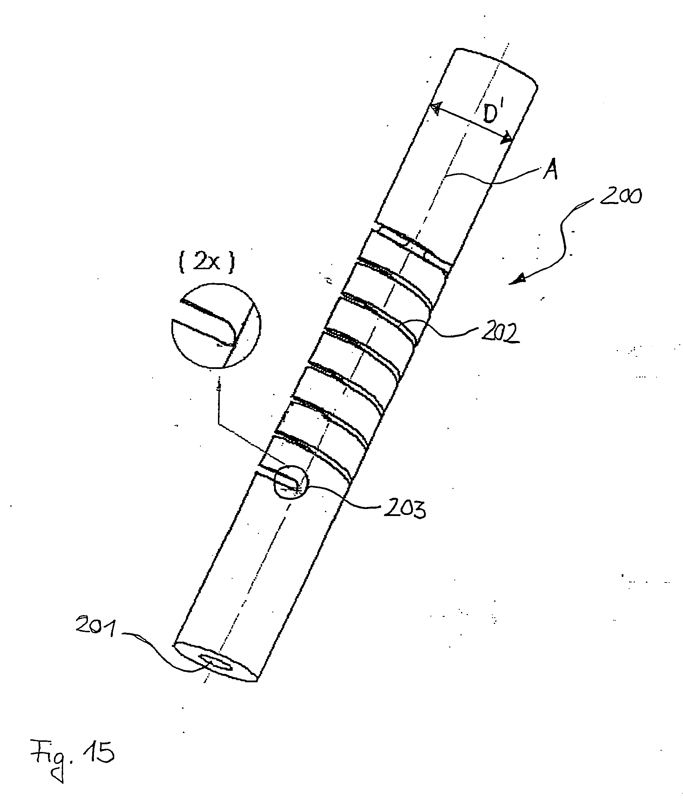

Alternativ zum Fräsen wird das Federelement 200 mit Drahterodieren, Laserbearbeitung oder Wasserstrahlbearbeitung aus dem zylinderförmigen Körper hergestellt. Hierbei wird wie in Fig. 15 gezeigt wiederum von einem Zylinder mit vorbestimmten Aussendurchmesser D' ausgegangen, in den dann im nächsten Schritt eine Bohrung 201 entlang der Hauptachse A über die gesamte Länge des Zylinderkörpers gebildet wird. Dann wird in die Wand des so gebildeten Hohlzylinders entlang der Hauptachse die Wand entlang einer Spirale 202 je nach Wandstärke des Hohlzylinders durch eines der oben angesprochenen Verfahren geschnitten. Der Auslauf 203 der Spirale 202 wird als Viertelkreis gebildet, sodass eine Nachbearbeitung des Auslaufs 203 in einem weiteren Arbeitsschritt gegenüber dem Fräsen entfällt. Auch ein Entgraten ist bei diesem Herstellungsverfahren nicht notwendig. Die Form des Auslaufs hat nicht zwingend die Form eines Viertelkreises, sondern kann auch jede andere beliebige Form, wie die eines anderen Kreisabschnittes, haben, durch die Belastungsspitzen in dem Material im Betrieb gering gehalten werden.

Schließlich wird wie bei dem Herstellungsverfahren mittels Fräsen in den zwei Endabschnitten der Bohrung 2 jeweils ein Innengewinde 4, 4' gebildet.

In einer Abwandlung wird bei den obigen Verfahren anstelle zumindest eines der Innengewindes 4, 4' zu Beginn des Verfahrens durch Drehen ein zylinderförmiger Ansatz mit einem Aussengewinde gebildet. In diesem Fall muß der Durchmesser der Bohrung kleiner als der Durchmesser des zylinderförmigen Ansatzes sein.

In einer weiteren Abwandlung des Herstellungsverfahrens wird das Federelement entsprechend der sechsten und siebten Ausführungsform ohne eine durchgehende Bohrung hergestellt.

Als Materialien für das Federelement, den Kern oder der damit verbundenen Elemente sind körperfreundliche Materialien, wie z.B. ein körperfreundliches Metall, wie Titan oder ein körperfreundlicher Kunststoff möglich. Auch die Verwendung einer Formgedächtnislegierung mit bekannten superelastischen Eigenschaften, z.B. Nitinol, ist möglich.

## Patentansprüche

1. Elastisches Element (72) zur Verwendung in einer
Stabilisierungseinrichtung (130) für Knochen oder Wirbel,
welches als ein rohrförmiger, im wesentlichen zylindrischer Körper mit einer spiralförmigen Ausnehmung (202) in seiner Wandung entlang seiner Hauptachse (A) mit einem ersten Ende und einem diesem gegenüberliegenden zweiten Ende und einer sich von dem ersten zum zweiten Ende erstreckenden koaxialen Bohrung (201) ausgebildet ist, wobei die Ausnehmung (202) in radialer Richtung in die Bohrung (201) des Körpers mündet, der an seinen gegenüberliegenden Enden jeweils entweder ein Innengewinde (4, 4', 41, 163) oder einen zylindrischen Ansatz mit einem Außengewinde (161, 171, 172) zum Verbinden mit einem Schaft und/oder einem Kopf einer Knochenschraube oder zum Verbinden mit einem Stababschnitt oder einer Platte aufweist, wobei
in der Bohrung (201) ein Kern (71) vorgesehen ist, der derart gestaltet ist, dass die Biegesteifigkeit des elastischen Elements erhöht wird, **dadurch gekennzeichnet daß**
der Kern (71) als Anschlag bei auf das elastiche Element ausgeübten Druckkräften dient.

2. Elastisches Element nach Anspruch 1, wobei das elastische Element aus einer Formgedächtnislegierung besteht.

3. Elastisches Element nach Anspruch 1, wobei das Element aus einem körperverträglichen Material, insbesondere aus Titan ausgebildet ist.

4. Elastisches Element nach einem der Ansprüche 1 bis 3, wobei der Körper an zumindest dem einen seiner gegenüberliegenden Enden einen zylindrischen Ansatz mit dem Außengewinde (161, 171, 172) aufweist.

5. Elastisches Element nach Anspruch 4, wobei das andere der seiner gegenüberliegenden Enden das Innengewinde (4, 4', 41, 163) aufweist.

6. Elastisches Element nach einem der vorhergehenden Ansprüche, wobei der Kern (641) einen Querschnitt besitzt, insbesondere rechteckig, oval oder asymmetrisch, welcher die Einstellung der Biegesteifigkeit des elastischen Abschnitts in einer bestimmten Richtung erlaubt.

7. Verwendung des elastischen Elements nach einem der Ansprüche 1 bis 6 in einem stabförmigen Element, welches zum Verbinden von zwei Knochenverankerungselementen dient, wobei das stabförmige Element einen ersten starren Stababschnitt aufweist und der erste Stababschnitt mit dem einen Ende des elastischen Elements verbunden wird.

8. Verwendung nach Anspruch 7, wobei ein zweiter starrer Stababschnitt mit dem anderen Ende des elastischen Elements verbunden wird.

9. Verwendung des elastischen Elements nach einem der Ansprüche 1 bis 6 in einer Platte, wobei die Platte einen zylinderförmigen Ansatz mit einem Außengewinde oder eine Bohrung mit einem Innengewinde an zumindest einem Ende der Platte aufweist und das eine Ende des elastischen Elements mit dem zylinderförmigen Ansatz mit dem Außengewinde oder der Bohrung mit dem Innengewinde verbunden wird.

10. Verfahren zur Herstellung eines elastischen Elementes, welches nach einem der Ansprüche 1 bis 6 ausgebildet ist, mit den Schritten:
(a) Bereitstellen eines zylinderförmigen Körpers;
(b) Bilden von jeweils einem zylinderförmigen Ansatz mit einem Außengewinde an den beiden Enden des zylinderförmigen Körpers durch spanabtragendes Drehen;
(c) Bilden einer spiralförmigen Ausnehmung durch von außen spanendes Abtragen von Material entlang einer zur Hauptachse des zylinderförmigen Körpers koaxialen Spirale; und
(d) Bilden einer Bohrung entlang der Hauptachse des zylinderförmigen Körpers, wobei die spiralförmige Ausnehmung in die Bohrung mündet;
(e) Einlegen eines Kerns in den zylinderförmigen Körper, wobei der Kern (71) als Anschlag bei auf das elastische Element ausgeübten Druckkräften dient.

11. Verfahren zum Herstellen eines elastischen Elements, welches nach einem der Ansprüche 1 bis 6 ausgebildet ist, mit den Schritten:
(a) Bereitstellen eines zylinderförmigen Körpers mit einem ersten und einem zweiten Ende;
(b) Bilden eines zylinderförmigen Ansatzes mit einem Durchmesser der kleiner ist als der vorbestimmte Außendurchmesser des in Schritt (a) bereitgestellten zylinderförmigen Körpers durch spanabtragendes Drehen und Bilden eines Außengewindes auf der Oberfläche des zylinderförmigen Ansatzes an dem ersten und an dem zweiten Ende des zylinderförmigen Körpers; und
(c) Schneiden einer Ausnehmung entlang einer zur Hauptachse des zylinderförmigen Körpers koaxialen Spirale mittels Drahterodieren, Laserbearbeiten oder Wasserstrahlbearbeiten;
(d) Bilden einer Bohrung entlang der Hauptachse des zylinderförmigen Körpers, wobei die spiralförmige Ausnehmung in die Bohrung mündet; und
(e) Einlegen eines Kerns in den zylinderförmigen Körper, wobei der Kern (71) als Anschlag bei auf das elastische Element ausgeübten Druckkräften dient.

## Claims

1. Elastic element (72) for use in a stabilization device (130) for bones or vertebrae,
which is formed as a tubular, substantially cylindrical body having a helical recess (202) formed in its wall along its main axis (A), a first end and a second end opposite the first end and a coaxial bore (201) extending from the first end to the second end,
wherein the recess (202) opens into the bore (201) of the body in a radial direction, which has at its opposite ends either an inner thread (4, 4', 41, 163) or a cylindrical boss having an outer thread (161, 171, 172) for connection with a shaft and/or a head of a bone screw, or for connection with a rod portion or a plate, wherein
a core (71) is provided within the bore (201), which is formed such as to increase a bending stiffness of the elastic element,
**characterized in that**
the core (71) serves as a stop upon exertion of pressure forces on the elastic element.

2. Elastic element according to claim 1, wherein the elastic element consists of a shape memory alloy.

3. Elastic element according to claim 1, wherein the element if formed from a body compatible material, in particular from titanium.

4. Elastic element according to one of claims 1 to 3, wherein the body has at at least one of its opposite ends a cylindrical boss having the outer thread (161, 171, 172).

5. Elastic element according to claim 4, wherein the other of the opposite ends comprises the inner thread (4, 4', 41, 163).

6. Elastic element according to one of the preceding claims, wherein the core (641) has a cross-section, in particular rectangular, oval or asymmetrical, which allows an adjustment of the bending stiffness of the elastic section in a predetermined direction.

7. Use of the elastic element according to one of claims 1 through 6 in a rod-shaped element, which serves for connecting two bone anchoring elements, wherein the rod-shaped element comprises a first rigid rod section and the first rod section is connected with the one end of the elastic element.

8. Use according to claim 7, wherein a second rigid rod section is connected with the other end of the elastic element.

9. Use of the elastic element according to one of claims 1 through 6 in a plate, wherein the plate comprises a cylindrically shaped boss having an outer thread or a bore having an inner thread at at least one end of the plate, and the other end of the elastic element is connected with the cylindrically shaped boss having the outer thread or with the bore having the inner thread.

10. Method of manufacturing an elastic element, which is formed according to one of claims 1 through 6, comprising the steps:
(a) providing a cylindrically shaped body;
(b) forming each a cylindrically shaped boss having an outer thread at both ends of the cylindrically shaped body by chip removing turning;
(c) forming a helically shaped recess by removing material from outside along a helix coaxial with the main axis of the cylindrically shaped body; and
(d) forming a bore along the main axis of the cylindrically shaped body wherein the helically shaped recess opens into the bore;
(e) inserting a core into the cylindrically shaped body, wherein the core (71) serves as a stop upon exertion of pressure forces on the elastic element.

11. Method of manufacturing an elastic element, which is formed according to one of claims 1 through 6, comprising the steps:
(a) providing a cylindrically shaped body;
(b) forming a cylindrically shaped boss having a diameter smaller than the predetermined outer diameter of the cylindrically shaped body provided in step (a) chip removing turning and forming an outer thread at the surface of the cylindrically shaped boss at the first and at the second end of the cylindrically shaped body; and
(c) cutting a recess along a helix coaxial with the main axis of the cylindrically shaped body via wire eroding, laser treatment or water-jet treatment;
(d) forming a bore along the main axis of the cylindrically shaped body wherein the helically shaped recess opens into the bore; and
(e) inserting a core into the cylindrically shaped body, wherein the core (71) serves as a stop upon exertion of pressure forces on the elastic element.

## Revendications

1. Elément élastique (72) destiné à être utilisé dans un dispositif de stabilisation (130) pour des os ou des vertèbres,
ledit élément élastique étant réalisé en tant que corps tubulaire sensiblement cylindrique avec un évidement hélicoïdal (202) dans sa paroi le long de son axe principal (A), avec une première extrémité et une deuxième extrémité opposée à celle-ci, et avec un alésage coaxial (201) s'étendant de la première à la deuxième extrémité, où l'évidement (202) débouche dans l'alésage (201) du corps en direction radiale, ledit corps présentant à chacune de ses extrémités opposées soit un filet femelle (4, 4', 41, 163), soit un embout cylindrique avec un filet mâle (161, 171, 172) pour une connexion avec la tige et/ou la tête d'une vis pour ostéosynthèse ou pour une connexion avec un tronçon de barre ou une plaque, où
une âme (71) est prévue dans l'alésage (201) et conformée de manière à accroître la résistance à la flexion de l'élément élastique,
**caractérisé**
**en ce que** l'âme (71) sert de butée pour les efforts de pression exercés sur l'élément élastique.

2. Elément élastique selon la revendication 1, où l'élément élastique est constitué d'un alliage à mémoire de forme.

3. Elément élastique selon la revendication 1, où l'élément est réalisé à partir d'un matériau à tolérance corporelle, en particulier en titane.

4. Elément élastique selon l'une des revendications 1 à 3, où le corps présente un embout cylindrique avec le filet mâle (161, 171, 172) sur au moins la première extrémité de ses extrémités opposées.

5. Elément élastique selon la revendication 4, où la deuxième extrémité de ses extrémités opposées présente le filet femelle (4, 4', 41, 163).

6. Elément élastique selon l'une des revendications précédentes, où l'âme (641) présente une section transversale, en particulier rectangulaire, ovale ou asymétrique, laquelle permet d'ajuster la résistance à la flexion et/ou la résistance à la torsion du tronçon élastique dans une direction définie.

7. Utilisation de l'élément élastique selon l'une des revendications 1 à 6 dans un élément en forme de barre servant à la connexion de deux éléments d'ancrage osseux, où l'élément en forme de barre présente un premier tronçon de barre rigide, et où le premier tronçon de barre peut être raccordé à la première extrémité de l'élément élastique.

8. Utilisation selon la revendication 7, où un deuxième tronçon de barre rigide peut être raccordé à la deuxième extrémité de l'élément élastique.

9. Utilisation de l'élément élastique selon l'une des revendications 1 à 6 dans une plaque, où la plaque présente un embout cylindrique avec un filet mâle ou un alésage avec un filet femelle sur au moins une extrémité de la plaque, et où la première extrémité de l'élément élastique est raccordée à l'embout cylindrique avec le filet mâle ou à l'alésage avec le filet femelle.

10. Procédé de fabrication d'un élément élastique réalisé selon l'une des revendications 1 à 6, comportant les étapes de :
(a) préparation d'un corps cylindrique ;
(b) formation d'un embout cylindrique avec un filet mâle à chacune des deux extrémités opposées du corps cylindrique par tournage avec enlèvement de copeaux ;
(c) formation d'un évidement hélicoïdal par enlèvement de matière depuis l'extérieur le long d'une spirale coaxiale à l'axe principal du corps cylindrique ; et
(d) formation d'un alésage le long de l'axe principal du corps cylindrique, l'évidement hélicoïdal débouchant dans l'alésage ;
(e) insertion d'une âme dans le corps cylindrique, l'âme (71) servant de butée pour les efforts de pression exercés sur l'élément élastique.

11. Procédé de fabrication d'un élément élastique réalisé selon l'une des revendications 1 à 6, comportant les étapes de:
(a) préparation d'un corps cylindrique avec une première et une deuxième extrémité ;
(b) formation d'un embout cylindrique de diamètre inférieur au diamètre extérieur prédéfini du corps cylindrique préparé en étape (a) par tournage avec enlèvement de copeaux, et formation d'un filet mâle à la surface de l'embout cylindrique sur la première et la deuxième extrémité du corps cylindrique ; et
(c) découpe d'un évidement le long d'une spirale coaxiale à l'axe principal du corps cylindrique par électroérosion à l'aide d'un fil, usinage laser ou usinage par jet d'eau ;
(d) formation d'un alésage le long de l'axe principal du corps cylindrique, l'évidement hélicoïdal débouchant dans l'alésage ; et
(e) insertion d'une âme dans le corps cylindrique, l'âme (71) servant de butée pour les efforts de pression exercés sur l'élément élastique.
